(19) **Europäisches Patentamt**
**European Patent Office**
**Office européen des brevets**

(11) **EP 2 450 031 B1**

(12) **EUROPEAN PATENT SPECIFICATION**

(45) Date of publication and mention
of the grant of the patent:
**29.08.2018 Bulletin 2018/35**

(51) Int Cl.:
*A61K 9/127* (2006.01)  *A61K 8/14* (2006.01)
*A61K 9/10* (2006.01)  *A61K 9/113* (2006.01)
*A61K 9/14* (2006.01)  *A61K 47/34* (2017.01)
*A61K 47/36* (2006.01)  *A61K 47/42* (2017.01)
*A61Q 19/00* (2006.01)

(21) Application number: **10793951.4**

(22) Date of filing: **02.06.2010**

(86) International application number:
**PCT/JP2010/059371**

(87) International publication number:
**WO 2011/001780 (06.01.2011 Gazette 2011/01)**

(54) **METHOD FOR PRODUCING LIPOSOMES BY TWO-STAGE EMULSIFICATION METHOD USING OUTER AQUEOUS PHASE CONTAINING SPECIFIC DISPERSING AGENT, METHOD FOR PRODUCING LIPOSOME DISPERSION OR DRY POWDER THEREOF USING THE METHOD FOR PRODUCING LIPOSOMES, AND LIPOSOME DISPERSION OR DRY POWDER THEREOF PRODUCED THEREBY**

VERFAHREN ZUR HERSTELLUNG VON LIPOSOMEN DURCH EIN ZWEISTUFEN-EMULGIERVERFAHREN UNTER VERWENDUNG DER ÄUSSEREN WÄSSRIGEN PHASE MIT SPEZIFISCHEN DISPERGIERMITTELN, VERFAHREN ZUR HERSTELLUNG DER LIPOSOM-DISPERSION ODER TROCKENPULVER DURCH VERWENDUNG DES VERFAHRENS ZUR HERSTELLUNG VON LIPOSOMEN UND LIPOSOMDISPERSION ODER TROCKENPULVER DARAUS

MÉTHODE DE PRODUCTION DE LIPOSOMES PAR UNE MÉTHODE D'ÉMULSIFICATION EN DEUX ÉTAPES EN UTILISANT UNE PHASE AQUEUSE EXTERNE CONTENANT UN AGENT DE DISPERSION SPÉCIFIQUE, MÉTHODE DE PRODUCTION D'UNE DISPERSION LIPOSOMIQUE OU D'UNE POUDRE SÈCHE CORRESPONDANTE EN UTILISANT LA MÉTHODE DE PRODUCTION DES LIPOSOMES ET DISPERSION LIPOSOMIQUE OU POUDRE SÈCHE CORRESPONDANTE PRODUITE DE CETTE MANIÈRE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **02.07.2009 JP 2009157626**

(43) Date of publication of application:
**09.05.2012 Bulletin 2012/19**

(73) Proprietor: **Konica Minolta Holdings, Inc.**
**Tokyo 100-0005 (JP)**

(72) Inventors:
• **MOTOKUI, Yasuyuki**
**Hino-shi**
**Tokyo 191-8511 (JP)**

• **WADA, Takeshi**
**Hino-shi**
**Tokyo 191-8511 (JP)**
• **ISODA, Takeshi**
**Hino-shi**
**Tokyo 191-8511 (JP)**

(74) Representative: **Gille Hrabal**
**Brucknerstrasse 20**
**40593 Düsseldorf (DE)**

(56) References cited:
**WO-A1-01/36583**  **WO-A1-99/25319**
**WO-A1-2005/053643**  **WO-A1-2008/108324**
**WO-A1-2010/110118**  **JP-A- 2001 181 213**
**JP-A- 2003 001 097**  **JP-A- 2006 272 196**

- **TAKAYUKI OHWAKI ET AL. INTERNATIONAL JOURNAL OF PHARMACEUTICS vol. 85, 1992, pages 19 - 28, XP023725478**

## Description

Technical Field

The present invention relates to a process for producing liposomes, a liposome dispersion or a dry powder of the dispersion by a two-step emulsification method, said liposomes, liposome dispersion or dry powder of the dispersion being used in the fields of medicines, cosmetics, foods, etc., or relates to a liposome dispersion or a dry powder thereof produced by a two-step emulsification method.

Background Art

Liposomes are each a closed vesicle composed of a lipid bilayer membrane of a single layer or plural layers, and can hold, in its inner aqueous phase and inside the lipid bilayer membrane, water-soluble drugs and the like and hydrophobic drugs and the like, respectively. Moreover, since the lipid bilayer membrane of the liposomes is analogous to a biomembrane, the liposomes have high safety in vivo. Therefore, various uses, such as medicines for DDS (drug delivery system), have been paid attention, and studies and development thereof have been promoted.

As one process for producing liposomes, a process using a two-step emulsification method is known, and for example, in a non patent literature 1, it is described that in the preparation of a W/O/W emulsion by a microchannel emulsification method using a W/O emulsion as a dispersion phase and using a tris-hydrochloric acid buffer solution as an outer aqueous phase, sodium caseinate was introduced as an emulsifying agent into the outer aqueous phase, whereby the encapsulation ratio of calcein in liposomes (lipid capsules) could be increased to about 80%.

A method for scalable, continuous flow production of a nucleic acid-containing microparticle has been invented. The microparticle produced by means of this method maintains the structural integrity of the associated nucleic acid and have a purity, which is suitable for introduction into an animal (e. g., human) host for delivery of a nucleic acid for gene therapy, antisense therapy, vaccination, treatment of autoimmune disease, and either specific or non-specific modulation of an immune response (see patent document 1).

A liposome encapsulating a water-soluble substance in an internal cavity of the liposome, which has particle size of 300 nm or less and contains triglycerol, has been reported. The water-soluble substance is a water-soluble low molecular weight compound and a polysaccharide. (see patent document 2).

Citation List

Patent Literature

    Patent Document 1: International Patent Application Publication WO 01/36583 A1
    Patent Document 2: International Patent Application Publication WO 2005/053643

Non Patent Literature

Non patent literature 1: Takashi Kuroiwa, Mitsutoshi Nakajima and Sosaku Ichikawa, "Influences of Aqueous Phase Composition in Lipid Capsule Formation Using Multiphase Emulsion as Base", Summary of the 74th Annual meeting of the Society of Chemical Engineers, Japan (March, 2009)

Summary of Invention

Technical Problem

Although sodium caseinate used in the invention described in the non patent literature 1 is a general substance as a food additive (stabilizer, emulsifying agent), it is not used as an additive of injections and is a substance having a fear of a problem of antigenicity. Moreover, it has been found that because sodium caseinate exists in the outer aqueous phase of liposomes for a long period of time, its influences on the lipid bilayer membranes of the liposomes are actualized, and leakage of an encapsulated drug is accelerated. Therefore, it is desirable to remove such a substance as much as possible after preparation of liposomes. However, sodium caseinate forms molecular aggregates (submicelles) having a volume mean particle diameter of about 15 nm in an aqueous solvent, and the molecular aggregates are associated with one another to form particles having a volume mean particle diameter of about 100 to 200 nm. Such molecular aggregates or such particles formed from the associated molecular aggregates have a volume mean particle diameter close to that of medical liposomes such as injections, and hence, there is a problem that it is difficult to separate and remove them from the prepared liposome dispersion.

It is an object of the present invention to provide a process for producing liposomes, a liposome dispersion or a dry powder of the dispersion by a two-step emulsification method using an additive (dispersing agent) by which a liposome dispersion and a dry powder thereof capable of inhibiting leakage of an encapsulated drug or the like from liposomes even in the long-term storage and capable of being stably used over a long period of time are obtained, and to provide a liposome dispersion and a dry powder thereof produced by the production process.

Solution to Problem

The present inventors have found that when specific dispersing agents, such as polysaccharides and gelatin, are added to an outer aqueous phase of the secondary emulsification step, these substances become favorable dispersing agents capable of contributing to long-term stabilization of liposomes or a liposome aqueous solution, and they have accomplished the present invention. The present inventors have also found that separation and removal of the specific dispersing agents after the formation of liposomes can further contribute to long-term stabilization of liposomes or a liposome dispersion.

That is to say, the process for producing liposomes of the present invention is a process for producing liposomes by a two-step emulsification method having a primary emulsification step to obtain a primary emulsification product, a secondary emulsification step to emulsify the primary emulsification product and an outer aqueous phase and a solvent removal step, wherein the outer aqueous phase in the secondary emulsification step contains a dispersing agent which forms no molecular self-aggregate or a dispersing agent which exclusively forms molecular self-aggregates having a volume mean particle diameter of not more than 10 nm (said dispersing agent being referred to as a "specific dispersing agent" hereinafter).

The weight-average molecular weight of the specific dispersing agent is preferably not less than 1,000 but not more than 100,000. The specific dispersing agent preferably contains at least one of protein, polysaccharides, an ionic surface active agent and a nonionic surface active agent, for example, at least one of gelatin, albumin, dextran and a polyalkylene oxide-based compound.

The volume mean particle diameter of the liposomes is preferably not less than 50 nm but not more than 300 nm.

As the emulsification method of the secondary emulsification step, a stirring emulsification method, a microchannel emulsification method or a membrane emulsification method using a SPG membrane is preferably used.

The liposomes are preferably unilamellar liposomes. As substances to be encapsulated in the liposomes, a drug or the like for medical treatments are preferably used.

Such a process for producing liposomes by a two-step emulsification method can be used for a process for producing a liposome dispersion or a dry powder thereof by combining it, if necessary, with a separation step for separating liposomes obtained by the secondary emulsification step and the specific dispersing agent from each other.

The liposome dispersion or the dry powder thereof of the present invention is characterized by being produced by such a production process as above, and may contain at least one of gelatin, albumin, dextran and a polyalkylene oxide-based compound.

Advantageous effects of Invention

By the production process of the present invention using a specific dispersing agent, liposomes, a liposome dispersion or a dry powder of the dispersion, which can inhibit leakage of an encapsulated drug or the like from liposomes even in the long-term storage and are preferable for medicines or the like having long-term stability, can be efficiently produced.

Description of Embodiments

Substances used in the production of liposomes

- Specific dispersing agent

In the present invention, an outer aqueous phase containing a "specific dispersing agent" (dispersing agent which forms no molecular self-aggregate or dispersing agent which exclusively forms molecular self-aggregates having a volume mean particle diameter of not more than 10 nm) is used in the secondary emulsification step.

In the present invention, the outer aqueous phase containing a "dispersing agent which forms no molecular self-aggregate" indicates both a case where a substance which forms no molecular aggregate (typically, micelles), however high the concentration may be increased is added as a dispersing agent to the outer aqueous phase, and a case where a substance which forms molecular aggregates in a certain concentration or higher (typically, surface active agent having a critical micelle concentration) is added as a dispersing agent to the outer aqueous phase in a concentration lower than said certain concentration. The outer aqueous phase containing a "dispersing agent which exclusively forms molecular self-aggregates having a volume mean particle diameter of not more than 10 nm" indicates a case where a substance which can form molecular aggregates but the molecular aggregates formed by which has a volume mean particle diameter of not more than 10 nm is added as a dispersing agent to the outer aqueous phase.

It is thought that the specific dispersing agents may be broadly classified into two types from the viewpoint of function. Dispersing agents of one type are those which are distributed all over the outer aqueous phase (W2) because of relatively low orientation to the interface between the primary emulsification product (W1/O) and the outer aqueous phase (W2) and have a function to stabilize liposomes by inhibiting adhesion of W1/O/W2 to one another, such as polysaccharides. Dispersing agents of the other type are those which have relatively high orientation to the interface of the W1/O/W2 emulsion and have a function to stabilize liposomes by surrounding the emulsion like a protective colloid, such as protein and nonionic surface active agents.

If W1/O/W2 are united to one another to form particles of larger diameters, removal of solvent by in-liquid drying is non-uniformly carried out, the encapsulated drug is liable to leak, etc., that is, liposomes become unstable. However, the specific dispersing agent prevents such uniting of W1/O/W2 and formation of particles of larger diameters and can inhibit formation of unstable liposomes, so that the specific dispersing agent contributes to enhancement of efficiency in the formation of unilamellar liposomes and to enhancement of the encapsulation ratio of the drug. If the specific dispersing agent is orientated to the interface of the W1/O/W2 emulsion, individual liposomes tend to get untied when the liposomes are formed with removal of the solvent, so that the specific dispersing agent also contributes to enhancement of efficiency in the formation of unilamellar liposomes and to enhancement of the encapsulation ratio of the drug.

As for the types of liposomes, several classification methods are known, and it is the main stream to discuss liposomes based on the following three types, so that those three types are used also in the present invention. The term "multivesicular liposomes (MVL) " means artificial fine lipid vesicles comprising a lipid membrane surrounding plural non-concentrically circular inner aqueous phases. On the other hand, "multilamellar liposomes (MLV)" have plural concentrically circular membranes like "coats of onion", and among them, there are shell-like concentrically circular aqueous compartments. The feature of the multivesicular liposomes and the multilamellar liposomes is that the volume mean particle diameter thereof is in the range of micrometers and is usually in the range of 0.5 to 25 $\mu$m. The term "unilamellar liposomes" used in the present invention has the same meaning as that of "mononuclear liposomes, and they have liposome structures having a single inner aqueous phase and usually have a volume mean particle diameter of about 20 to 500 nm.

The specific dispersing agent exerts a given function in the formation of liposomes, as described above, and the mixed lipid component constituted mainly of phospholipid having hydration ability has self-organizing ability, so that even if no dispersing agent is present after the formation of liposomes, the dispersed state can be maintained.

Typical examples of the specific dispersing agents in the present invention include protein, polysaccharides, ionic surface active agents and nonionic surface active agents. When the specific dispersing agent is a substance approved as an additive in medicines, etc. (substance guaranteed to have no serious influence on the human body even if it is administered into the body), there is no substantial clinical problem even if a part of it remains in the liposome dispersion after a filtration step.

Examples of the proteins include gelatin (soluble protein obtained by denaturing collagen through heating), albumin and trypsin. Gelatin usually has a molecular weight of several thousands to several millions, and for example, gelatin having a weight-average molecular weight of 1,000 to 100,000 is preferable. Gelatin commercially available as that for medical treatments or foods can be used. Examples of the albumins include ovalbumin (molecular weight: about 45,000), serum albumin (molecular weight: about 66,000, bovine serum albumin) and lactalbumin (molecular weight: about 14,000, $\alpha$-lactalbumin). For example, dry desugared albumen that is ovalbumin is preferable.

Examples of the polysaccharides include dextran, starch, glycogen, agarose, pectin, chitosan, carboxylmethyl cellulose sodium, xanthan gum, locust beam gum, guar gum, maltotriose, amylose, pullulan, heparin and dextrin. For example, dextran having a weight-average molecular weight of 1,000 to 100,000 is preferable.

Examples of the ionic surface active agents include sodium cholate and sodium deoxycholate.

Examples of the nonionic surface active agents include alkyl glucoxides, such as octyl glucoxide, polyalkylene oxide-based compounds, such as products of "Tween 80" (available from Tokyo Kasei Industry Co. Ltd., polyoxyethylene sorbitan monooleate, molecular weight: 1309.68) and "Pluronic F-68" (available from BASF, polyoxyethylene (160) polyoxypropylene (30) glycol, number-average molecular weight: 9600), and polyethylene glycols having a weight-average molecular weight of 1000 to 100000. Examples of products of the polyethylene glycols (PEG) include "Unilube" (available from NOF Corporation), GL4-400NP and GL4-800NP (available from NOF Corporation), PEG 200,000 (available fromWakoPure Chemical Industries, Ltd.) andMacrogoal (available from Sanyo Chemical Industries, Ltd.).

Whether the specific dispersing agent forms molecular self-aggregates in the outer aqueous phase or not, or whether the volume mean particle diameter of the molecular aggregates formed is not more than 10 nm or not (that is, whether the substance added to the outer aqueous phase satisfies requirements for the specific dispersing agent or not) can be confirmed by, for example, a particle size distribution meter of dynamic light scattering type or a freeze fracturing method using a transmission electron microscope (TEM).

The amount of the specific dispersing agent added to the outer aqueous phase (concentration of the specific dispersing agent) is controlled in an appropriate range according to the type of the dispersing agent. When a substance which forms molecular self-aggregates (having a volume mean particle diameter of more than 10 nm) in a certain concentration is added as the specific dispersing agent, the amount added is controlled in a range wherein the concentration is less than the certain concentration. If the concentration is too high, troubles are sometimes brought about in the measurement based on the particle size distribution system depending upon the type of the dispersing agent, and therefore, it is preferable to control the concentration in a range of low concentrations wherein such troubles are not brought about.

Taking into consideration separation between liposomes and the specific dispersing agent in the filtration step, the volume mean particle diameter of the molecular self-aggregates of the specific dispersing agent or assemblies thereof is preferably not more than 1/10, more preferably not more than 1/100, the volume mean particle diameter of the liposomes.

If the molecular weight of the specific dispersing agent is too low, there is a fear that the specific dispersing agent is liable to be incorporated into the lipid membrane to inhibit formation of liposomes. On the other hand, if the molecular weight thereof is too high, there is a fear that the rate of dispersion of the W1/O/W2 emulsion in the outer aqueous phase or the rate of orientation to the interface is lowered to cause uniting of liposomes or formation of multivesicular liposomes. On this account, the weight-average molecular weight of the specific dispersing agent is preferably not less than 1,000 but not more than 100,000. When the weight-average molecular weight is in this range, the encapsulation ratio of the drug in the liposomes is good.

- Mixed lipid components (F1) and (F2)

The mixed lipid component (F1) used in the primary emulsification step mainly constitutes an inner membrane of the lipid bilayer membrane of liposomes. The mixed lipid component (F2) mainly forms an outer membrane. The mixed lipid components (F1) and (F2) may have the same compositions or different compositions.

The compositions of these mixed lipid components are not specifically restricted, but in general, they are mainly constituted of phospholipids (e.g. , lecithin derived from animals and plants; phosphatidylcholine, phosphatidylserine, phosphatidylglycerol, phosphatidylinositol, phosphatidic acid, and glycerophospholipids that are fatty acid esters thereof; sphingolipid; and derivatives thereof) and sterols that contribute to stabilization of lipid membrane (e.g., cholesterol, phytosterol, ergosterol, and derivatives thereof). In addition, glycolipid, glycol, aliphatic amine, long-chain aliphatic acids (e.g., oleic acid, stearic acid, and palmitic acid), and other compounds that impart other various functions may be added. In the present invention, neutral phospholipids, such as dipalmitoyl phosphatidylcholine (DPPC) and dioleyl phosphatidylcholine (DOPC), are commonly used as the phospholipids. When F2 contains a lipid component necessary for impartation of a function of DDS, such as PEG phospholipid, efficient modification of the liposome surfaces becomes possible. The blending ratio between the mixed lipid components is appropriately controlled according to the intended use, taking into consideration stability of the lipid membrane and properties of liposomes such as behaviors thereof in vivo.

- Aqueous solvents (W1) and (W2), organic solvent (O)

As the aqueous solvents (W1) and (W2) and the organic solvent (O), publicly known general ones can be used. The aqueous solvent (W1) and the organic solvent (O) used in the primary emulsification step form an aqueous phase and an oil phase of a W1/O emulsion, respectively, and the aqueous solvent (W2) used in the secondary emulsification step forms an outer aqueous phase of a W1/O/W2 emulsion. The aqueous solvents are, for example, aqueous solvents obtained by blending pure water with other solvents compatible with water, salts or saccharides for adjusting osmotic pressure, buffer solutions for adjusting pH, etc., if necessary. The organic solvents are, for example, organic solvents composed of compounds incompatible with aqueous solvents, such as hexane (n-hexane) and chloroform. An organic solvent containing hexane as a main component (not less than 50% by volume) is preferable because monodispersibility of the resulting W1/O emulsion of nano size is good.

- Substances to be encapsulated

In the present invention, the substances (referred to as "drugs and the like" generically) to be encapsulated in the liposomes are not specifically restricted, and various substances that are known in the fields of medicines, cosmetics, foods, etc. can be used according to the intended use of the liposomes.

Examples of water-soluble drugs and the like for medical treatments among drugs and the like include substances having pharmacological actions, such as contrast media (nonionic iodine compound for X-ray contrast radiography, complex composed of gadolinium and chelating agent for MRI contrast radiography, etc.), antitumor agents (adriamycin, pirarubicin, vincristine, taxol, cisplatin, mitomycin, 5-fluorouracil, irinotecan, estracyt, epirubicin, carboplatin, intron, Gemzar, methotrexate, cytarabine, Isovorin, tegafur, etoposide, Topotecin, nedaplatin, cyclophosphamide, melphalan, ifosfamide, Tespamin, nimustine, ranimustine, dacarbazine, enocitabine, fludarabine,pentostatin, cladribine, daunomycin, aclarubicin, amrubicin, actinomycin, taxotere, trastuzumab, rituximab, gemtuzumab, lentinan, sizofiran, interferon, intrleukin, asparaginase, fostestrol, busulfan, bortezomib, Alimta, bevacizumab, nelarabine, cetuximab, etc.), antibacterial agents (macrolide antibiotics, ketolide antibiotics, cephalosporin antibiotics, oxacephem antibiotics, penicillin antibiotics, $\beta$-lactamase agents, aminoglycoside antibiotics, tetracycline antibiotics, fosfomycin antibiotics, carbapenem antibiotics, penem antibiotics), MRSA·VRE·PRSP infectious disease remedies, polyene antifungal agents, pyrimidine antifungal agents, azole antifungal agents, candin antifungal agents, new quinolone synthetic antibacterial agents, antioxidants, anti-inflammatory agents, blood circulation accelerating agents, whitening agents, skin roughness preventing agents, anti-aging agents, hair growth accelerating agents, moisture retention agents, hormone agents, vitamins, nucleic acid (sense strand or anti-sense strand of DNA or RNA, plasmid, vector, mRNA, siRNA, etc.), proteins (enzyme, antibody, peptide, etc.), and vaccine formulations (those having toxides of tetanus and the like as antigens; those having viruses of diphtheria, Japanese encephalitis, poliomyelitis, rubella, mumps, hepatitis and the like as antigens; DNA or RNA vaccine; etc.), and formulation assisting agents, such as dyes/fluorescent dyes (calcein), chelating agents, stabilizers and preservatives.

## Process for producing liposomes

The process for producing liposomes by a two-step emulsification method according to the present invention has the following steps (1) to (3). This production process forms liposomes in the outer aqueous phase containing a specific dispersing agent, and therefore, it naturally becomes a process for producing a liposome dispersion. Moreover, by appropriately combining it with a separation step (4) and other steps (5) such as a dry-powdering step, if necessary, a process for producing a liposome dispersion or a dry powder thereof is obtained.

(1) Primary emulsification step

The primary emulsification step is a step of emulsifying the organic solvent (O), the aqueous solvent (W1) and the mixed lipid component (F1) to prepare a W1/O emulsion.

The preparation process for a W1/O emulsion is not specifically restricted, and the preparation can be carried out by the use of apparatuses, such as ultrasonic emulsifier, stirring emulsifier, membrane emulsifier and high-pressure homogenizer. For the membrane emulsification, a premix membrane emulsification method wherein a W1/O emulsion of large particle diameters is prepared in advance and then the emulsion is passed through a membrane of small pore diameters to prepare a W1/O emulsion of smaller particle diameters may be used.

The pH value of the aqueous solvent (W1) is usually in the range of 3 to 10, and can be adjusted in a preferred range according to the mixed lipid component. For example, when oleic acid is used as the mixed lipid component, the pH of the aqueous solvent (W1) is preferably in the range of 6 to 8.5. For the pH adjustment, an appropriate buffer solution is used.

The volume mean particle diameter of the W1/O emulsion, the proportion of the mixed lipid component (F1) added to the organic solvent (O), the volume ratio between the organic solvent (O) and the aqueous solvent (W1), and other operation conditions in the primary emulsification step can be properly controlled according to the emulsification method adopted, taking into consideration the conditions of the subsequent secondary emulsification step, the type of the finally prepared liposomes, etc. In usual, the proportion of the mixed lipid component (F1) to the organic solvent (O) is in the range of 1 to 50% by mass, and the volume ratio between the organic solvent (O) and the aqueous solvent (W1) is in the range of 100:1 to 1:2.

In the present invention, in order to encapsulate a water-soluble drug or the like in liposomes, any of a method (i) in which a water-soluble drug or the like is dissolved or suspended in the aqueous solvent (W1) of the primary emulsification step in advance, and at the time of completion of the secondary emulsification step, liposomes encapsulating the drug or the like are obtained, and a method (ii) in which (empty) liposomes encapsulating no water-soluble drug or the like is first obtained, then a water-soluble drug or the like is added to the dispersion of the liposomes, or when a freeze-dried powder once obtained is redispersed in an aqueous solvent, a water-soluble drug or the like is added, and the mixture is stirred to incorporate the drug or the like into the liposomes may be used. Fat-soluble drugs or the like can be also encapsulated in the liposomes by adding them in advance in the primary emulsification step as in the method (i) or by adding them after obtaining empty liposomes as in the method (ii).

(2) Secondary emulsification step

The secondary emulsification step is a step to prepare a W1/O/W2 emulsion using the W1/O emulsion obtained by the step (1) .

In the present invention, an outer aqueous phase (W2) containing the specific dispersing agent is used in this secondary emulsification step. In usual, an aqueous solvent for forming the outer aqueous phase and the specific dispersing agent are mixed to prepare the outer aqueous phase (W2) in advance, and the W1/O emulsion is dispersed therein.

The method to prepare the W1/O/W2 emulsion in the secondary emulsification step is not specifically restricted, and a membrane emulsification method (emulsification method using SPG membrane, or the like), a microchannel emulsification method, a stirring emulsification method, a droplet method, a contact method, etc. can be used, and preferable are a membrane emulsification method, a microchannel emulsification method and a stirring emulsification method. The microchannel emulsification method and the membrane emulsification method using a SPG membrane are characterized in that a W1/O/W2 emulsion of uniform particle diameters can be prepared as compared with other emulsification methods, and they are preferable from the viewpoint that collapse of droplets and leakage of an encapsulated substance from droplets in the emulsification operation can be suppressed because mechanical shear force is not necessary for the emulsification. A terrace length, a channel depth and a channel width of the microchannel substrate and a pore diameter of the SPG membrane can be properly controlled according to the size of the W1/O/W2 emulsion to be formed, but for example, the pore diameter of the SPG membrane is usually in the range of 0.1 to 100 $\mu$m.

In the membrane emulsification method, a membrane permeation method such as a premix membrane emulsification method wherein a W1/O/W2 emulsion of large particle diameters is prepared in advance and then the emulsion is passed through a membrane of small pore diameters to prepare a W1/O/W2 emulsion of smaller particle diameters may be used. The premix membrane emulsification method is preferable because energy required is low, the throughput is large, and preparation of liposomes can be sped up.

In the stirring emulsification, techniques and apparatuses used for mixing two or more fluids can be used. For example, there are stirring apparatuses in various shapes . There are many apparatuses in which a stirrer in the form of a bar, a plate or a propeller is rotated at a fixed rate in one direction in a tank, but in some apparatuses, a stirrer is subjected to intermittent rotation or reverse rotation. In special circumstances, various devices, such that plural stirrers are arranged and alternately rotated reversely, and a protrusion or a plate combined with a stirrer is mounted on a tank side to increase shear stress generated by the stirrer, are made. For the power transmission to the stirrer, there are various means, and in most apparatuses, the stirrer is rotated via a rotating shaft, but there is a magnetic stirrer in which power is transmitted to a stirrer encasing a magnet therein and coated with Teflon (registered trade mark) or the like from the outside of the container by means of a rotating magnetic field.

Moreover, there are apparatuses for a fluid having low viscosity in which no stirrer is used and a fluid or external air is pressurized with a pump installed outside a tank to vigorously blow it into the tank and thereby stir the interior of the tank, such as an aeration apparatus for a small aquarium tank and an industrial spray drying apparatus. Examples of milling machines called mills include hammer mill, pin mill, angmill, CoBall mill, apex mill, ball mill, jet mill, roll mill, colloid mill and dispersion mill. These are machines to mix fluids by the action of mechanical force, such as compression force, pressing force, expansion force, shear force, impact force or cavitation force. In addition to such mechanical methods, electrical stirring methods can be also used.

The mode (order of addition, or the like) of mixing the aqueous solvent (W2), the W1/O emulsion, the mixed lipid component (F2) and the specific dispersing agent is not specifically restricted, and an appropriate mode is selected. For example, when the F2 is mainly composed of a water-soluble lipid, the F2 and the specific dispersing agent are added to W2 in advance, and to the mixture is added the W1/O emulsion, whereby emulsification can be carried out. On the other hand, when the F2 is mainly composed of a fat-soluble lipid, a W1/O emulsion is prepared in advance, then the F2 is added to the oil phase of the W1/O emulsion, and the mixture is added to W2 to which the specific dispersing agent has been added, whereby emulsification can be carried out.

The volume mean particle diameter of the W1/O/W2 emulsion, the proportion of the mixed lipid component (F2) added to the aqueous solvent (W2) or the organic solvent (O) of the W1/O emulsion, the volume ratio between the W1/O emulsion and the aqueous solvent (W2), the amount of the specific dispersing agent added, and other operation conditions in the secondary emulsification step can be properly controlled, taking into consideration the use purpose of the finally prepared liposomes, etc.

(3) Solvent removal step

The solvent removal step is a step of removing the organic solvent phase (O) contained in the W1/O/W2 emulsion obtained by the secondary emulsification step, to form a dispersion of liposomes. Examples of methods for removing the solvent include an evaporation method using an evaporator and an in-liquid drying method.

The in-liquid drying method is a method in which the W1/O/W2 emulsion is recovered, transferred into an open container and allowed to stand still or stirred, whereby the organic solvent (O) contained in the W1/O/W2 emulsion is evaporated and removed. Through such operations, a lipid membrane composed of the mixed lipid components (F1) and (F2) is formed around the inner aqueous phase, and a dispersion of liposomes can be obtained. In this case, removal of the solvent by distillation can be accelerated by heating or pressure reduction. The temperature conditions and the pressure reduction conditions are properly controlled in a conventional manner according to the type of the organic solvent used. The temperature is set in a range wherein the solvent does not undergo bumping, and for example, the temperature is preferably in the range of 0 to 60°C, more preferably 0 to 25°C. The pressure is preferably set in the range of a saturated vapor pressure of the solvent to the atmospheric pressure, and more preferably set in the range of a saturated vapor pressure of the solvent + 1% to a saturated vapor pressure of the solvent + 10%. When a mixture of different solvents is used, conditions fitted to a solvent of a higher saturated vapor pressure are preferable. These removal conditions may be combined in a range wherein the solvent does not undergo bumping. For example, when a drug having low resistance to heat is used, the solvent is preferably distilled off under the conditions of a lower temperature and reduced pressure. By stirring the W1/O/W2 emulsion during the solvent removal, the solvent removal proceeds more uniformly. The step (2) and the step (3) may be carried out continuously. For example, the W1/O/W2 emulsion is prepared by a stirring method in the step (2), and then stirring is further continued to remove the solvent.

In the liposomes obtained by the production process of the present invention, multivesicular liposomes derived from the W1/O/W2 emulsion are sometimes contained in a certain proportion. In order to lower the proportion, it is effective to carry out stirring or pressure reduction, preferably a combination of them. It is important to carry out pressure reduction and stirring for a longer period of time than that required for removal of most of the solvent. It is thought that by virtue of this, hydration of the lipid constituting the liposomes proceeds, whereby the multivesicular liposomes are untied and separated into unilamellar liposomes. Moreover, it is surprising that even if these operations are carried out, leakage of the encapsulated substance does not occur. When multivesicular liposomes remain even after such operations are carried out, the multivesicular liposomes can be removed by a filter utilizing a difference in particle diameter.

The volume mean particle diameter of the liposomes finally obtained by such a production process as above (the later-described granulation step may be used, when needed), is not specifically restricted, but when the liposomes are used as liposome formulations for medical treatments, the volume mean particle diameter thereof is preferably not less than 50 nm but not more than 1,000 nm, more preferably not less than 50 nm but nor more than 300 nm. The liposomes of such sizes hardly choke blood capillaries and can pass through gaps formed in blood vessels in the vicinity of cancer tissues, so that they are advantageously used when administered to the human body as medicines.

(4) Separation step

The separation step is a step of separating the specific dispersing agent and liposomes from each other to remove the specific dispersing agent from the liposome dispersion. For example, if a microfiltration membrane (MF membrane, pore diameter: about 50 nm to 10 $\mu$m) or an ultrafiltration membrane (UF membrane, pore diameter: about 2 to 20 nm) having a specific pore diameter is used, the liposomes can be efficiently separated from the specific dispersing agent that has formed molecular self-aggregates (volume mean particle diameter of, for example, not more than 10 nm). When there is no problem in view of the use purpose of the product even if the specific dispersing agent is not separated from the liposomes, this separation step does not have to be arranged. By arranging the separation step, leakage of the encapsulated drug or the like from liposomes can be further inhibited, and liposomes having long-term stability can be formed.

(5) Other steps

Examples of other steps that are carried out when needed include granulation step and dry-powdering step.

Through the granulation step, the particle diameters of the liposomes prepared can be adjusted to be in the desired range. For example, by the use of a hydrostatic extruder (e.g., "Extruder" manufactured by Nichiyu Liposome Co., Ltd., "Liponizer" manufactured by Nomura Micro Science Co., Ltd.) equipped with a polycarbonate membrane or a cellulose membrane having a pore diameter of 0.1 to 0.4 $\mu$m as a filter, liposomes having a central particle diameter of about 50 to 500 nm are efficiently obtained. If the above "Extruder" or the like is used, multivesicular liposomes secondarily formed from the W1/O/W2 emulsion can be untied into unilamellar liposomes.

It is also desirable that the liposome dispersion is dry-powdered by freeze drying or the like to make its form suitable for storage before use. The freeze drying can be carried out using the same means or device as used in the production of conventional liposomes. The freeze drying is carried out under the appropriate conditions (temperature: -120 to -20°C, pressure: 1 to 15 Pa, time: 16 to 26 hours, etc.) in accordance with, for example, indirect heating freezing method, refrigerant direct expansion method, heating medium circulation method, triple heat exchange method or redundant refrigeration method. By introducing the freeze-dried product obtained as above into water, a dispersion of liposomes can be prepared again.

Examples

(Measuring method for volume mean particle diameter)

The volume mean particle diameter of liposomes in the examples and the comparative examples described below was measured by the following method.

The W1/O emulsion was diluted with a chloroform/hexane mixed solvent (volume ratio: 4/6, the specific gravity was made equal to that of the inner aqueous phase) to 10 times, then a particle size distribution was measured with a dynamic light scattering nanotrack particle size analyzer (UPA-EX150, manufactured by Nikkiso Co., Ltd.), and based on this particle size distribution, a volume mean particle diameter was calculated.

As for the volume mean particle diameter of the specific dispersing agent which forms molecular aggregates, a liposome dispersion (also referred to as a "suspension" hereinafter) prepared in each of the following examples was subjected to the measurement as it was, using the same device. That is to say, a suspension of liposome particles prepared was diluted with a phosphoric acid buffer saline solution (PBS) to 25 times, then a particle size distribution was measured using a dynamic light scattering nanotrack particle size analyzer (UPA-EX150, manufactured by Nikkiso Co., Ltd.), and based on this particle size distribution, a volume mean particle diameter was calculated.

Example 1

(Production of W1/O emulsion by primary emulsification step)

15 ml of hexane containing 0.3 g of egg yolk lecithin "COATSOME NC-50" (available from NOF Corporation) having a phosphatidylcholine content of 95%, 0.152 g of cholesterol (Chol) and 0.108 g of oleic acid (OA) was used as an organic solvent phase (O), and 5 ml of a tris-hydrochloric acid buffer solution (pH: 8, 50 mmol/L) containing calcein (0.4 mM) was used as an aqueous dispersion phase (W1) for an inner aqueous phase. A mixed liquid of them was placed in a 50 ml beaker and irradiated with ultrasonic waves (output: 5.5) at 25°C for 15 minutes using an ultrasonic dispersion device (UH-600S, manufactured by MST Co., Ltd.) in which a probe having a diameter of 20 mm had been set, to carry out emulsification. As a result of the aforesaid measurement, the W1/O emulsion obtained in this primary emulsification step was confirmed to be a monodisperse W/O emulsion having a volume mean particle diameter of about 220 nm.

(Production of W1/O/W2 emulsion by secondary emulsification step)

Subsequently, the W1/O emulsion obtained by the above primary emulsification step was used as a dispersion phase, and production of a W1/O/W2 emulsion by a microchannel emulsification method was carried out by the use of a laboratory dead-end type microchannel emulsification device module.

The microchannel substrate of the module was a substrate made of silicon, and a terrace length, a channel depth and a channel width of the microchannel substrate were about 60 $\mu$m, about 11 $\mu$m and about 16 $\mu$m, respectively. A glass plate was compression bonded to the microchannel substrate to form a channel. The outlet side of the channel was filled with a tris-hydrochloric acid buffer solution (pH: 8, 50 mmol/L) containing alkali-treated gelatin (isoelectric point: about 5) of 3%, which was an outer aqueous phase solution (W2), and the W1/O emulsion was fed through an inlet of the channel to produce a W1/O/W2 emulsion.

(Production of liposomes by removal of organic solvent phase)

Next, the W1/O/W2 emulsion was transferred into an open glass container without a lid and stirred with a stirrer at room temperature for about 20 hours to evaporate hexane. Thus, a suspension of fine liposome particles was obtained, and it was confirmed that calcein was contained in the particles.

Example 2

Production was carried out in the same manner as in Example 1, except that the dispersing agent of the outer aqueous phase was changed to "Tween 80" (available from Tokyo Kasei Industry Co. Ltd., polyoxyethylene sorbitan monooleate, molecular weight: 1309.68) from the alkali-treated gelatin and the concentration thereof was changed to 1% in the production of W1/O/W2 emulsion by secondary emulsification step. As a result, a suspension of fine liposome particles was obtained, and it was confirmed that calcein was contained in the particles.

Example 3

Production was carried out in the same manner as in Example 1, except that the dispersing agent of the outer aqueous phase was changed to albumin (available from Kewpie Corporation, alias: dry desugared egg white) from the alkali-treated gelatin in the production of W1/O/W2 emulsion by secondary emulsification step. As a result, a suspension of fine liposome particles was obtained, and it was confirmed that calcein was contained in the particles.

Example 4

Production was carried out in the same manner as in Example 1, except that the dispersing agent of the outer aqueous phase was changed to carboxydextran from the alkali-treated gelatin in the production of W1/O/W2 emulsion by secondary emulsification step. As a result, a suspension of fine liposome particles was obtained, and it was confirmed that calcein was contained in the particles.

Example 5

Production was carried out in the same manner as in Example 1, except that the dispersing agent of the outer aqueous phase was changed to purified gelatin (available fromNippi Incorporated, Nippi high grade gelatin type AP) from the alkali-treated gelatin in the production of W1/O/W2 emulsion by secondary emulsification step. As a result, a suspension of fine liposome particles was obtained, and it was confirmed that calcein was contained in the particles.

Example 6

Production was carried out in the same manner as in Example 5, except that the emulsification method was changed to a membrane emulsification method using a SPG membrane from the microchannel emulsification method in the production of W1/O/W2 emulsion by secondary emulsification step. As a result, a suspension of fine liposome particles was obtained, and it was confirmed that calcein was contained in the particles. That is to say, the W1/O emulsion obtained by the primary emulsification step was used as a dispersion phase, and production of a W1/O/W2 emulsion by a SPG membrane emulsification method was carried out. In a SPG membrane emulsification device (manufactured by SPG Technology Co., Ltd., trade name "External Pressure Type Micro Kit"), a cylindrical SPG membrane having a diameter of 10 mm, a length of 20 mm and a pore diameter of 2.0 $\mu$m was used. The outlet side of the device was filled with a tris-hydrochloric acid buffer solution (pH: 8, 50 mmol/L) containing purified gelatin (available from Nippi Incorporated, Nippi high grade gelatin type AP), which was an outer aqueous phase solution (W2), and the W1/O emulsion was fed through an inlet of the device to produce a W1/O/W2 emulsion. The pressure required for the membrane emulsification was about 25 kPa.

Example 7

Production was carried out in the same manner as in Example 5, except that the emulsification method was changed to a stirring emulsification method from the microchannel emulsification method in the production of W1/O/W2 emulsion by secondary emulsification step. As a result, a suspension of fine liposome particles was obtained, and it was confirmed that calcein was contained in the particles. That is to say, in the stirring emulsification, the W1/O emulsion was fed to the W2 vigorously stirred with a stirrer, whereby a W1/O/W2 emulsion was produced.

Example 8

Production was carried out in the same manner as in Example 1, except that the dispersing agent of the outer aqueous phase was changed to sodium cholate (molecular weight: 430) from the alkali-treated gelatin and the concentration thereof was changed to 0.1% in the production of W1/O/W2 emulsion by secondary emulsification step. As a result, a suspension of fine liposome particles was obtained, and it was confirmed that calcein was contained in the particles.

Example 9

Production was carried out in the same manner as in Example 6, except that the dispersing agent of the outer aqueous phase was changed to sodium cholate (molecular weight: 430) from the purified gelatin and the concentration thereof was changed to 0.1% in the production of W1/O/W2 emulsion by secondary emulsification step. As a result, a suspension of fine liposome particles was obtained, and it was confirmed that calcein was contained in the particles.

### Example 10

Production was carried out in the same manner as in Example 7, except that the dispersing agent of the outer aqueous phase was changed to sodium cholate (molecular weight: 430) from the purified gelatin and the concentration thereof was changed to 0.1% in the production of W1/O/W2 emulsion by secondary emulsification step. As a result, a suspension of fine liposome particles was obtained, and it was confirmed that calcein was contained in the particles.

### Example 11

Production was carried out in the same manner as in Example 1, except that the dispersing agent of the outer aqueous phase was changed to octyl glucoside (molecular weight: 292) from the alkali-treated gelatin and the concentration thereof was changed to 1% in the production of W1/O/W2 emulsion by secondary emulsification step. As a result, a suspension of fine liposome particles was obtained, and it was confirmed that calcein was contained in the particles.

### Example 12

Production was carried out in the same manner as in Example 6, except that the encapsulated drug was changed to cytarabine from calcein. As a result, a suspension of fine liposome particles was obtained, and it was confirmed that cytarabine was contained in the particles.

### Example 13

Production was carried out in the same manner as in Example 7, except that the encapsulated drug was changed to cytarabine from calcein. As a result, a suspension of fine liposome particles was obtained, and it was confirmed that cytarabine was contained in the particles.

### Examples 14 to 23

Productions of Examples 14 to 23 were each carried out in the same manner as in Example 1, except that the dispersing agent of the outer aqueous phase was changed to a specific dispersing agent shown in Table 1 from the alkali-treated gelatin in the production of W1/O/W2 emulsion by secondary emulsification step. As a result, suspensions of fine liposome particles were obtained, and it was confirmed that calcein was contained in the particles.

### Example 24

Production was carried out in the same manner as in Example 23, except that the emulsification method was changed to a membrane emulsification method using a SPG membrane from the microchannel emulsification method in the production of W1/O/W2 emulsion by secondary emulsification step. As a result, a suspension of fine liposome particles was obtained, and it was confirmed that calcein was contained in the particles.

### Example 25

Production was carried out in the same manner as in Example 23, except that the emulsification method was changed to a stirring emulsification method from the microchannel emulsification method in the production of W1/O/W2 emulsion by secondary emulsification step. As a result, a suspension of fine liposome particles was obtained, and it was confirmed that calcein was contained in the particles.

### Examples 26 to 30

Productions of Examples 26 to 30 were each carried out in the same manner as in Example 1, except that the dispersing agent of the outer aqueous phase was changed to a specific dispersing agent shown in Table 1 from the alkali-treated gelatin in the production of W1/O/W2 emulsion by secondary emulsification step. As a result, suspensions of fine liposome particles were obtained, and it was confirmed that calcein was contained in the particles.

### Example 31

Production was carried out in the same manner as in Example 30, except that the emulsification method was changed to a membrane emulsification method using a SPG membrane from the microchannel emulsification method in the production of W1/O/W2 emulsion by secondary emulsification step. As a result, a suspension of fine liposome particles was obtained, and it was confirmed that calcein was contained in the particles.

Example 32

Production was carried out in the same manner as in Example 2, except that the emulsification method was changed to a membrane emulsification method using a SPG membrane from the microchannel emulsification method in the production of W1/O/W2 emulsion by secondary emulsification step. As a result, a suspension of fine liposome particles was obtained, and it was confirmed that calcein was contained in the particles.

Examples 33 to 35

Productions of Examples 33 to 35 were each carried out in the same manner as in Example 32, except that the dispersing agent of the outer aqueous phase was changed to a specific dispersing agent shown in Table 1 from Tween 80 in the production of W1/O/W2 emulsion by secondary emulsification step. As a result, suspensions of fine liposome particles were obtained, and it was confirmed that calcein was contained in the particles.

Example 36

Production was carried out in the same manner as in Example 1, except that the encapsulated drug was changed to cytarabine from calcein. As a result, a suspension of fine liposome particles was obtained, and it was confirmed that cytarabine was contained in the particles.

Example 37

Production was carried out in the same manner as in Example 2, except that the encapsulated drug was changed to cytarabine from calcein. As a result, a suspension of fine liposome particles was obtained, and it was confirmed that cytarabine was contained in the particles.

Example 38

Production was carried out in the same manner as in Example 3, except that the encapsulated drug was changed to cytarabine from calcein. Asaresult, a suspension of fine liposome particles was obtained, and it was confirmed that cytarabine was contained in the particles.

Example 39

Production was carried out in the same manner as in Example 4, except that the encapsulated drug was changed to cytarabine from calcein. As a result, a suspension of fine liposome particles was obtained, and it was confirmed that cytarabine was contained in the particles.

Example 40

Production was carried out in the same manner as in Example 5, except that the encapsulated drug was changed to cytarabine from calcein. Asaresult, a suspension of fine liposome particles was obtained, and it was confirmed that cytarabine was contained in the particles.

Comparative Example 1

(Production of W1/O emulsion by primary emulsification step)

15 ml of hexane containing 0.3 g of egg yolk lecithin "COATSOME NC-50" (available from NOF Corporation) having a phosphatidylcholine content of 95%, 0.152 g of cholesterol (Chol) and 0.108 g of oleic acid (OA) was used as an organic solvent phase (O), and 5 ml of a tris-hydrochloric acid buffer solution (pH: 8, 50 mmol/L) containing calcein (0.4 mM) was used as an aqueous dispersion phase (W1) for an inner aqueous phase. A mixed liquid of them was placed in a 50 ml beaker and irradiated with ultrasonic waves (output: 5.5) at 25°C for 15 minutes using an ultrasonic dispersion device (UH-600S, manufactured by MST Co. , Ltd.) in which a probe having a diameter of 20 mm had been set, to carry out emulsification. As a result of the aforesaid measurement, the W1/O emulsion obtained in this primary emulsification step was confirmed to be a monodisperse W/O emulsion having a volume mean particle diameter of about 220 nm.

(Production of W1/O/W2 emulsion by secondary emulsification step)

Subsequently, the W1/O emulsion obtained by the above primary emulsification step was used as a dispersion phase, and production of a W1/O/W2 emulsion by a microchannel emulsification method was carried out by the use of a laboratory dead-end type microchannel emulsification device module.

The microchannel substrate of the module was a substrate made of silicon, and a terrace length, a channel depth and a channel width of the microchannel substrate were about 60 $\mu$m, about 11 $\mu$m and about 16 $\mu$m, respectively. A glass plate was compression bonded to the microchannel substrate to form a channel. The outlet side of the channel was filled with a tris-hydrochloric acid buffer solution (pH: 8, 50 mmol/L) containing sodium caseinate of 3%, which was an outer aqueous phase solution (W2), and the W1/O emulsion was fed through an inlet of the channel to produce a W1/O/W2 emulsion.

(Production of liposomes by removal of organic solvent phase)

Next, the W1/O/W2 emulsion was transferred into an open glass container without a lid and stirred with a stirrer at room temperature for about 20 hours to evaporate hexane. Thus, a suspension of fine liposome particles was obtained, and it was confirmed that calcein was contained in the particles.

Comparative Example 2

Production was carried out in the same manner as in Comparative Example 1, except that the tris-hydrochloric acid buffer solution (pH: 8, 50 mmol/L) containing sodium caseinate of 3%, which was an outer aqueous phase solution (W2), was replaced with a tris-hydrochloric acid buffer solution (pH: 8, 50 mmol/L) in the production of W1/O/W2 emulsion by secondary emulsification step. As a result, a W1/O/W2 emulsion was once formed but immediately suffered uniting of W1/O/W2 with one another, and consequently, a stable W1/O/W2 emulsion was not obtained. On this account, experiment of the next step could not be carried out.

Comparative Example 3

Production was carried out in the same manner as in Comparative Example 1, except that the tris-hydrochloric acid buffer solution (pH: 8, 50 mmol//L) containing sodium caseinate of 3%, which was an outer aqueous phase solution (W2), was replaced with a tris-hydrochloric acid buffer solution (pH: 8, 50 mmol/L) containing sodium dodecylbenzenesulfonate of 3% in the production of W1/O/W2 emulsion by secondary emulsification step. As a result, a suspension was obtained.

Comparative Example 4

Production was carried out in the same manner as in Comparative Example 1, except that the emulsification method was changed to a membrane emulsification method using a SPG membrane from the microchannel emulsification method in the production of W1/O/W2 emulsion by secondary emulsification step. As a result, a suspension of fine liposome particles was obtained, and it was confirmed that calcein was contained in the particles.

Comparative Example 5

Production was carried out in the same manner as in Comparative Example 4, except that the encapsulated drug was changed to cytarabine from calcein. As a result, a suspension of fine liposome particles was obtained, and it was confirmed that cytarabine was contained in the particles.

Comparative Example 6

Production was carried out in the same manner as in Comparative Example 1, except that the emulsification method was changed to a stirring emulsification method from the microchannel emulsification method in the production of W1/O/W2 emulsion by secondary emulsification step. As a result, a suspension of fine liposome particles was obtained, and it was confirmed that calcein was contained in the particles.

(Evaluation of stability of liposomes)

As for Examples 1 to 40 and Comparative Examples 1 to 6, encapsulation ratios at the time of formation of liposomes and after 1 month were determined, and stability of liposomes after 1 month was evaluated. The results are set forth in Table 1.

Stability of liposomes after 1 month was evaluated in the following manner using the following formula.

$$\texttt{Lowering ratio of encapsulation (\%) = 100 - (encapsulation}$$

$$\texttt{ratio after 1 month/encapsulation ratio at the time of formation}$$

$$\texttt{of liposomes)} \times \texttt{100}$$

　　　AA: The lowering ratio of encapsulation is not less than 0% but less than 5%.

　　　BB: The lowering ratio of encapsulation is not less than 5% but less than 15%.

　　　CC: The lowering ratio of encapsulation is not less than 15% but less than 35%.

　　　DD: The lowering ratio of encapsulation is not less than 35% but not more than 100%.

(Encapsulation ratio of liposomes (%))

As for the liposomes obtained in Examples 1 to 40 and Comparative Example 1 to 6, encapsulation ratios at the time of formation of liposomes and after 1 month were measured in accordance with the following method. The results are set forth in Table 1.

Encapsulation ratio measuring method in the case of using calcein as encapsulated substance

Total fluorescence intensity ($F_{total}$) of a suspension (3 mL) of liposome particles was measured with a spectrophotometer (U3310, manufactured by JASCO Corporation) . Next, 30 $\mu$l of a 0.01 M $CoCl_2$ tris-hydrochloric acid buffer solution was added to quench fluorescence of an encapsulated drug calcein which had leaked into the outer aqueous phase, by means of $Co^{2+}$, whereby fluorescence intensity ($F_{in}$) inside the liposomes was measured. Moreover, liposomes were produced under the same conditions as those for the sample, except that calcein was not added, and fluorescence ($F_l$) emitted by the lipid itself was measured. The encapsulation ratio was calculated from the following formula.

$$\texttt{Encapsulation ratio (\%)} = (F_{in}-F_l)/(F_{total}-F_l) \times 100$$

(Encapsulation ratio measuring method in the case of using cytarabine as encapsulated substance)

A suspension of liposome particles was subjected to component separation using an ultracentrifuge under the ultracentrifugal conditions, and the amount of cytarabine contained in the solids (liposomes) and the amount thereof contained in the supernatant solution were determined by HPLC (column: VarianPolaris C18-A (3 $\mu$m, 2×40mm)). The encapsulation ratio (%) of cytarabine was calculated in the following manner. That is to say, the determined value of the solids (liposomes), i.e., amount of cytarabine encapsulated in the liposomes, and the determined value of the supernatant solution, i.e., amount of cytarabine not contained in the liposomes, were totalized, then by the resulting total value, the amount of cytarabine contained in the liposomes was divided, and the resulting value was multiplied by 100.

(Liposomes after 1 month)

A suspension of liposomes particles was allowed to stand still in a constant-temperature vessel (20°C) for 1 month to store it.

Table 1

| | Dispersing agent | | | | Encapsulated substance | Secondary emulsification method | Volume mean particle diameter of liposomes (nm) | Encapsulation ratio of liposomes at the time of formation (%) | Encapsulation ratio after 1 month (%) | Stability |
| | Name of dispersing agent | Weight-average molecular weight | Molecular aggregate | Volume mean particle diameter (nm) | | | | | | |
|---|---|---|---|---|---|---|---|---|---|---|
| Ex. 1 | gelatin | 50000 | not formed | - | calcein | microchannel | 221 | 65 | 61 | BB |
| Ex. 2 | Tween 80 | 1309 | formed | 3 | calcein | microchannel | 249 | 50 | 47 | BB |
| Ex. 3 | albumin | 45000 | not formed | - | calcein | microchannel | 230 | 66 | 62 | BB |
| Ex. 4 | dextran | 40000 | not formed | - | calcein | microchannel | 209 | 53 | 50 | BB |
| Ex. 5 | purified gelatin | 8000 | not formed | - | calcein | microchannel | 231 | 68 | 63 | BB |
| Ex. 6 | purified gelatin | 8000 | not formed | - | calcein | SPG | 233 | 63 | 58 | BB |
| Ex. 7 | purified gelatin | 8000 | not formed | - | calcein | stirring | 197 | 59 | 53 | BB |
| Ex. 8 | sodium cholate | 430 | formed | 7 | calcein | microchannel | 261 | 9 | 8 | BB |
| Ex. 9 | sodium cholate | 430 | formed | 7 | calcein | SPG | 255 | 12 | 11 | BB |
| Ex. 10 | sodium cholate | 430 | formed | 7 | calcein | stirring | 221 | 8 | 7 | BB |
| Ex. 11 | octyl glucoside | 292 | formed | 6 | calcein | microchannel | 281 | 6 | 4 | CC |
| Ex. 12 | purified gelatin | 8000 | not formed | - | cytarabine | SPG | 240 | 42 | 38 | BB |
| Ex. 13 | purified gelatin | 8000 | not formed | - | cytarabine | stirring | 188 | 41 | 35 | BB |
| Ex. 14 | maltotriose | 504 | not formed | - | calcein | microchannel | 287 | 11 | 10 | BB |
| Ex. 15 | gelatin 600 | 600 | not formed | - | calcein | microchannel | 267 | 12 | 9 | CC |

| | Dispersing agent | | | | Encapsulated substance | Secondary emulsification method | Volume mean particle diameter of liposom es (nm) | Encapsulation ratio of 1 iposomes at the time of formation (%) | Encapsulation ratio after 1 month (%) | Stability |
|---|---|---|---|---|---|---|---|---|---|---|
| | Name of dispersing agent | Weight-average molecular weight | Molecular aggregate | Volume mean particle diameter (nm) | | | | | | |
| Ex. 16 | gelatin 2000 | 2000 | not formed | - | calcein | microchannel | 233 | 58 | 54 | BB |
| Ex. 17 | trypsin | 10000 | not formed | - | calcein | microchannel | 211 | 55 | 52 | BB |
| Ex. 18 | dextran 60000 | 60000 | not formed | - | calcein | microchannel | 252 | 60 | 50 | CC |
| Ex. 19 | pullulan | 47000 | not formed | - | calcein | microchannel | 179 | 53 | 50 | BB |
| Ex. 20 | low-molecular heparin | 5000 | not formed | - | calcein | microchannel | 221 | 58 | 54 | BB |
| Ex. 21 | γ-dextrin | 1297.12 | not formed | - | calcein | microchannel | 225 | 51 | 48 | BB |
| Ex. 22 | Unilube | 20000 | not formed | - | calcein | microchannel | 243 | 68 | 62 | BB |
| Ex. 23 | Pluronic | 9600 | not formed | - | calcein | microchannel | 249 | 70 | 66 | BB |
| Ex. 24 | Pluronic | 96000 | not formed | - | calcein | SPG | 214 | 70 | 66 | BB |
| Ex. 25 | Pluronic | 96000 | not formed | - | calcein | stirring | 177 | 68 | 64 | BB |
| Ex. 26 | Macrogoal 4000 | 4000 | not formed | - | calcein | microchannel | 203 | 53 | 50 | BB |
| Ex. 27 | NOF GL4-400NP | 40000 | not formed | - | calcein | microchannel | 200 | 59 | 52 | BB |
| Ex. 28 | NOF GL4-800NP | 80000 | not formed | - | calcein | microchannel | 227 | 50 | 46 | BB |
| Ex. 29 | PEG 200000 | 200000 | not formed | - | calcein | microchannel | 279 | 7 | 6 | BB |
| Ex. 30 | gelatin | 150000 | formed | - | calcein | microchannel | 288 | 9 | 7 | CC |
| Ex. 31 | gelatin | 50000 | not formed | - | calcein | SPG | 226 | 59 | 55 | BB |
| Ex. 32 | Tween 80 | 1309 | formed | 3 | calcein | SPG | 230 | 63 | 58 | BB |

17

(continued)

| | Dispersing agent | | | | Encapsulated substance | Secondary emulsification method | Volume mean particle diameter of liposomes (nm) | Encapsulation ratio of liposomes at the time of formation (%) | Encapsulation ratio after 1 month (%) | Stability |
| | Name of dispersing agent | Weight-average molecular weight | Molecular aggregate | Volume mean particle diameter (nm) | | | | | | |
|---|---|---|---|---|---|---|---|---|---|---|
| Ex. 33 | albumin | 45000 | not formed | - | calcein | SPG | 211 | 58 | 54 | BB |
| Ex. 34 | dextran | 40000 | not formed | - | calcein | SPG | 209 | 54 | 50 | BB |
| Ex. 35 | purified gelatin | 8000 | not formed | - | calcein | SPG | 223 | 60 | 56 | BB |
| Ex. 36 | gelatin | 50000 | not formed | - | cytarabine | microchannel | 255 | 32 | 30 | BB |
| Ex. 37 | Tween 80 | 1309 | formed | 3 | cytarabine | microchannel | 212 | 42 | 39 | BB |
| Ex. 38 | albumin | 45000 | not formed | - | cytarabine | microchannel | 233 | 38 | 34 | BB |
| Ex. 39 | dextran | 45000 | not formed | - | cytarabine | microchannel | 262 | 42 | 39 | BB |
| Ex. 40 | purified gelatin | 8000 | not formed | - | cytarabine | microchannel | 196 | 39 | 36 | BB |
| Comp. Ex. 1 | casein | 23000 | formed | 15 | calcein | micro channel | 230 | 94 | 60 | DD |
| Comp. Ex. 2 | none | - | - | - | calcein | microchannel | - | 0 (could not be prepared) | 0 | - |
| Comp. Ex. 3 | SDS | 288 | formed | 80 | calcein | microchannel | 209 | 8 | 4 | DD |
| Comp. Ex. 4 | casein | 23000 | formed | 15 | calcein | SPG | 224 | 78 | 46 | DD |
| Comp. Ex. 5 | casein | 23000 | formed | 15 | cytarabine | SPG | 222 | 33 | 17 | DD |
| Comp. Ex. 6 | casein | 23000 | formed | 15 | calcein | stirring | 267 | 65 | 40 | DD |

EP 2 450 031 B1

18

It can be seen from Table 1 that the liposomes produced by the use of the specific dispersing agent of the present invention hardly suffered lowering of the encapsulation ratio of the encapsulated drug even after one month and they were stable for a long period of time. On the other hand, it can be seen that the liposomes produced by the use of a substance other than the specific dispersing agent of the present invention suffered marked lowering of the encapsulation ratio and they lacked stability. The liposomes described in Table 1 were all unilamellar liposomes .

Examples 41 to 49

The suspensions of liposome particles produced in Examples 1, 2, 3, 4, 6, 11, 19, 23 and 30 were subjected to the later-described microfiltration/ultrafiltration step to produce liposome suspensions of Examples 41 to 49.

Comparative Examples 7 and 8

The suspensions of liposome particles produced in Comparative Examples 1 and 3 were subjected to the later-described microfiltration/ultrafiltration step to produce liposome suspensions of Comparative Examples 7 and 8.

(Microfiltration/ultrafiltration step)

Separation by microfiltration/ultrafiltration was carried out in the following manner. That is to say, a filter having an appropriately determined pore diameter was set in a pressure filtration device (dead-end type), and a liquid obtained by diluting the suspension of liposome particles with a tris-hydrochloric acid buffer solution (pH: 8, 50 mmol/L) to 10 times was subjected to filtration with the device. The amount of the dispersing agent contained in the filtrate was analyzed to examine whether the dispersing agent could be separated or not. A case where not less than 80% of the dispersing agent could be separated was expressed by "could be separated", and a case where only not more than 10% of the dispersing agent could be separated was expressed by "could be hardly separated". There was no case where the amount between them could be separated.

(Evaluation of stability of liposomes)

Stability of liposomes obtained in Examples 41 to 49 and Comparative Examples 7 and 8 was evaluated in the same manner as in the aforesaid evaluation of stability of liposomes. The results are set forth in Table 2.

Table 2

| | Dispersing agent | | | | Encapsulated substance | Secondary emulsification method | Volume mean particle diameter of liposomes (n m) | Encapsulation ratio of liposomes at the time of formation (%) | Encapsulation ratio after 1 month (%) (after separation of dispersing agent) | Separation by ultrafiltration/ microfiltration | Stability |
| | Name of dispersing agent | Weight - average molecular weight | Molecular aggregate | Volume mean particle diameter (nm) | | | | | | | |
|---|---|---|---|---|---|---|---|---|---|---|---|
| Ex. 41 | gelatin | 50000 | not formed | - | calcein | microchannel | 221 | 65 | 65 | could be separated | AA |
| Ex. 42 | Tween 80 | 1309 | formed | 3 | calcein | microchannel | 249 | 50 | 50 | could be separated | AA |
| Ex. 43 | albumin | 45000 | not formed | - | calcein | microchannel | 230 | 66 | 65 | could be separated | AA |
| Ex. 44 | dextran | 40000 | not formed | - | calcein | microchannel | 209 | 53 | 52 | could be separated | AA |
| Ex. 45 | purified gelatin | 8000 | not formed | - | calcein | SPG | 233 | 63 | 63 | could be separated | AA |
| Ex. 46 | octyl glucoside | 292 | formed | 6 | calcein | microchannel | 281 | 6 | 6 | could be separated | AA |
| Ex. 47 | pullulan | 47000 | not formed | - | calcein | microchannel | 179 | 53 | 52 | could be separated | AA |
| Ex. 48 | Pluronic | 9600 | no t formed | | calcein | microchannel | 249 | 70 | 69 | could be separated | AA |
| 49 | gelatin | 150000 | not formed | - | calcein | Ex. microchannel | 288 | 9 | 9 | could be separated | AA |
| Comp. Ex. 7 | casein | 23000 | formed | 15 | calcein | microchannel | 230 | 94 | 63 | could be hardly separated | cc |
| Comp. Ex. 8 | SDS | 288 | formed | 80 | calcein | microchannel | 209 | 8 | 4 | could be hardly separated | DD |

It can be seen from Table 2 that lowering of the encapsulation ratio after 1 month in the case of the liposome suspension obtained by separating the specific dispersing agent of the present invention from the suspension of liposome particles produced by the use of the specific dispersing agent was suppressed as compared with that in the case of the liposome suspension from which the specific dispersing agent had not been separated, and the lowering ratio was less than 5%. In the comparative examples using no specific dispersing agent of the present invention, separation and removal of the dispersing agent used could not been carried out, so that there was no change in the encapsulation ratio after 1 month though a separation step was carried out. The reason why separation and removal of the dispersing agent used could not been carried out is presumed to be that molecular self-aggregates were present. Actually, volume mean particle diameters of the solution containing only casein and the solution containing only SDS, said casein and said SDS being dispersing agents, were measured, and as a result, main particle size distributions of casein and SDS were observed at 15 nm and 80 nm, respectively. Further, a volume mean particle diameter of the solution containing only casein having a higher concentration was measured, and as a result, a particle size distribution thought to be that of an associated substance of molecular aggregates was observed at 150 nm in addition to the above particle size distribution at 15 nm. From this, it can be readily presumed that when casein of a high concentration is used for the production of liposomes, separation of casein from liposomes would be more difficult.

**Claims**

1. A process for producing unilamellar liposomes by two-step emulsification method having
   a primary emulsification step to obtain a W1/O emulsion by emulsifying an organic solvent (O), an aqueous solvent (W1) and a lipid component which constitutes the lipid membrane of liposomes,
   a secondary emulsification step to obtain a W1/O/W2 emulsion by emulsifying the W1/O emulsion obtained by the primary emulsification step and an aqueous solvent (W2) to be an outer aqueous phase, and
   a solvent removal step to remove the organic solvent phase from the W1/O/W2 emulsion obtained by the secondary emulsification step,
   **characterized by** that the outer aqueous phase in the secondary emulsification step contains a dispersing agent which forms no molecular self-aggregate or a dispersing agent which exclusively forms molecular self-aggregates having a volume mean particle diameter of not more than 10 nm, wherein the dispersing agent is selected from the group consisting of protein, polysaccharides, an ionic surface active agent and a nonionic surface active agent.

2. The process for producing unilamellar liposomes by a two-step emulsification method as claimed in claim 1, wherein the weight-average molecular weight of the dispersing agent is not less than 1,000 but not more than 100,000.

3. The process for producing unilamellar liposomes by a two-step emulsification method as claimed in claim 1 or 2, wherein the dispersing agent contains at least one of gelatin, albumin, trypsin, dextran, starch, glycogen, agarose, pectin, chitosan, carboxylmethyl cellulose sodium, xanthan gum, locust beam gum, guar gum, maltotriose, amylose, pullulan, heparin, dextrin, sodium cholate, sodium deoxycholate, an alkyl glucoside and a polyalkylene oxide-based compound.

4. The process for producing unilamellar liposomes by a two-step emulsification method as claimed in claim 3, wherein the dispersing agent contains at least one of gelatin, albumin, dextran and a polyalkylene oxide-based compound.

5. The process for producing unilamellar liposomes by a two-step emulsification method as claimed in any one of claims 1 to 4, wherein the volume mean particle diameter of the unilamellar liposomes is not less than 50 nm but not more than 300 nm.

6. The process for producing unilamellar liposomes by a two-step emulsification method as claimed in any one of claims 1 to 5, which uses a stirring emulsification method as the emulsification method of the secondary emulsification step.

7. The process for producing unilamellar liposomes by a two-step emulsification method as claimed in any one of claims 1 to 5, which uses a microchannel emulsification method as the emulsification method of the secondary emulsification step.

8. The process for producing unilamellar liposomes by a two-step emulsification method as claimed in any one of claims 1 to 5, which uses a membrane emulsification method using a SPG membrane as the emulsification method of the secondary emulsification step.

9. The process for producing unilamellar liposomes by a two-step emulsification method as claimed in any one of claims 1 to 8, wherein a drug for medical treatments are used as substances to be encapsulated in the unilamellar liposomes.

10. A process for producing a unilamellar liposome dispersion or a dry powder thereof, comprising the process for producing unilamellar liposomes by a two-step emulsification method as claimed in any one of claims 1 to 9.

11. The process for producing a unilamellar liposome dispersion or a dry powder thereof as claimed in claim 10, which further has a separation step to separate unilamellar liposomes obtained by the secondary emulsification step and the dispersing agent from each other.

**Patentansprüche**

1. Verfahren zur Herstellung unilamellarer Liposome mit Hilfe eines zweistufigen Emulgierverfahrens mit
einem primären Emulgierschritt zum Erhalt einer W1/O-Emulsion durch Emulgieren eines organischen Lösemittels (O), eines wasserhaltigen Lösemittels (W1) und einer Lipidkomponente, die die Lipidmembran für Liposome bildet, einem sekundären Emulgierschritt zum Erhalt einer W1/O/W2-Emulsion durch Emulgieren der durch den primären Emulgierschritt erhaltenen W1/O-Emulsion und eines wasserhaltigen Lösemittels (W2), das eine äußere wässrige Phase bildet, und
einem Schritt zum Entfernen des Lösemittels zur Abscheidung der organischen Lösemittelphase aus der durch den sekundären Emulgierschritt erhaltenen W1/O/W2-Emulsion,
**dadurch gekennzeichnet, dass** die äußere wässrige Phase in dem sekundären Emulgierschritt ein Dispersionsmittel enthält, das ein nicht molekulares Selbstaggregat bildet, oder ein Dispersionsmittel, das ausschließlich molekulare Selbstaggregate bildet, welche einen mittleren Volumenpartikeldurchmesser von nicht mehr als 10 nm aufweisen, wobei das Dispersionsmittel ausgewählt ist aus der Gruppe, die aus Protein, Polysacchariden, einem ionischen Tensid und einem nicht-ionischen Tensid besteht.

2. Verfahren zur Herstellung unilamellarer Liposome mit Hilfe eines zweistufigen Emulgierverfahrens gemäß Anspruch 1, wobei das gewichtsdurchschnittliche Molekulargewicht des Dispersionsmittels nicht weniger als 1000, aber nicht mehr als 100.000 beträgt.

3. Verfahren zur Herstellung unilamellarer Liposome mit Hilfe eines zweistufigen Emulgierverfahrens gemäß Anspruch 1 oder 2, wobei das Dispersionsmittel zumindest eine der Substanzen aus Gelatine, Albumin, Trypsin, Dextran, Stärke, Glykogen, Agarose, Pektin, Chitosan, Carboxylmethylcellulose-Natrium, Xanthan, Johannisbrotkernmehl, Guaran, Maltotriose, Amylose, Pullulan, Heparin, Dextrin, Natriumcholat, Natriumdeoxycholat, ein Alkylglukosid und eine auf Polyalkylenoxid basierende Verbindung enthält.

4. Verfahren zur Herstellung unilamellarer Liposome mit Hilfe eines zweistufigen Emulgierverfahrens gemäß Anspruch 3, wobei das Dispersionsmittel zumindest eine der Substanzen aus Gelatine, Albumin, Dextran und eine auf Polyalkylenoxid basierende Verbindung enthält.

5. Verfahren zur Herstellung unilamellarer Liposome mit Hilfe eines zweistufigen Emulgierverfahrens gemäß irgendeinem der Ansprüche 1 bis 4, wobei der mittlere Volumenpartikeldurchmesser der unilamellaren Liposome nicht weniger als 50 nm, aber nicht mehr als 300 nm beträgt.

6. Verfahren zur Herstellung unilamellarer Liposome mit Hilfe eines zweistufigen Emulgierverfahrens gemäß irgendeinem der Ansprüche 1 bis 5, welches ein Rühr-Emulgierverfahren als Emulgierverfahren des sekundären Emulgierschrittes einsetzt.

7. Verfahren zur Herstellung unilamellarer Liposome mit Hilfe eines zweistufigen Emulgierverfahrens gemäß irgendeinem der Ansprüche 1 bis 5, welches ein Mikrokanal-Emulgierverfahren als Emulgierverfahren des sekundären Emulgierschrittes einsetzt.

8. Verfahren zur Herstellung unilamellarer Liposome mit Hilfe eines zweistufigen Emulgierverfahrens gemäß irgendeinem der Ansprüche 1 bis 5, welches ein Membran-Emulgierverfahren unter Verwendung einer SPG-Membran als Emulgierverfahren des sekundären Emulgierschrittes einsetzt.

**9.** Verfahren zur Herstellung unilamellarer Liposome mit Hilfe eines zweistufigen Emulgierverfahrens gemäß irgendeinem der Ansprüche 1 bis 8, wobei Arzneimittel für medizinische Behandlungen als Substanzen verwendet werden, die in den unilamellaren Liposomen einzukapseln sind.

**10.** Verfahren zur Herstellung einer Dispersion mit unilamellaren Liposomen oder eines Trockenpulvers davon, mit dem Verfahren zur Herstellung unilamellarer Liposome mit Hilfe eines zweistufigen Emulgierverfahrens gemäß irgendeinem der Ansprüche 1 bis 9.

**11.** Verfahren zur Herstellung einer Dispersion mit unilamellaren Liposomen oder eines Trockenpulvers davon gemäß Anspruch 10, welches ferner einen Trennungsschritt zum Trennen der mit Hilfe des sekundären Emulgierschritts erhaltenen unilamellaren Liposome und des Dispersionsmittels voneinander aufweist.

**Revendications**

**1.** Procédé de production de liposomes unilamellaires par moyen d'un procédé d'émulsification en deux étapes comprenant :

une étape d'émulsification primaire pour obtenir une émulsion W1/O en émulsifiant un solvant organique (O), un solvant aqueux (W1) et un composant lipidique, qui constitue la membrane lipidique de liposomes, une étape d'émulsification secondaire pour obtenir une émulsion W1/O/W2 en émulsifiant l'émulsion W1/O obtenue par l'étape d'émulsification primaire et un solvant aqueux (W2) qui forme une phase aqueuse extérieure, et
une étape d'enlèvement de solvant pour enlever la phase de solvant organique dans l'émulsion W1/O/W2 obtenue par l'étape d'émulsification secondaire,
**caractérisé en ce que** la phase aqueuse extérieure dans l'étape d'émulsification secondaire contient un agent de dispersion, qui forme des agrégats autonomes non-moléculaires, ou un agent de dispersion, qui forme exclusivement des agrégats autonomes moléculaires ayant un diamètre moyen de particule de volume, qui n'est pas supérieur à 10 nm, l'agent de dispersion étant sélectionné dans le groupe composé de protéine, de polysaccharides, d'agent tensioactif ionique et d'agent tensioactif non ionique.

**2.** Procédé de production de liposomes unilamellaires par moyen d'un procédé d'émulsification en deux étapes selon la revendication 1, dans lequel le poids moléculaire moyen de l'agent de dispersion n'est pas inférieur à 1.000, mais pas supérieur à 100.000.

**3.** Procédé de production de liposomes unilamellaires par moyen d'un procédé d'émulsification en deux étapes selon la revendication 1 ou la revendication 2, dans lequel l'agent de dispersion contient au moins une substance parmi la gélatine, l'albumine, la trypsine, le dextran, l'amidon, le glycogène, l'agarose, la pectine, le chitosan, le sodium de carboxylméthylcellulose, la gomme xanthane, la farine de graines de caroube, la gomme de guar, le maltotriose, l'amylose, le pullulan, l'héparine, le dextrine, le cholate de sodium, le déoxycholate de sodium, un alkyl glucoside et un composé à base de polyalkylène oxyde.

**4.** Procédé de production de liposomes unilamellaires par moyen d'un procédé d'émulsification en deux étapes selon la revendication 3, dans lequel l'agent de dispersion contient au moins une substance parmi la gélatine, l'albumine, le dextran, et un composé à base de polyalkylène oxyde.

**5.** Procédé de production de liposomes unilamellaires par moyen d'un procédé d'émulsification en deux étapes selon l'une quelconque des revendications 1 à 4, dans lequel le diamètre moyen de particule de volume des liposomes unilamellaires n'est pas inférieur à 50 nm, mais pas supérieur à 300 nm.

**6.** Procédé de production de liposomes unilamellaires par moyen d'un procédé d'émulsification en deux étapes selon l'une quelconque des revendications 1 à 5, qui utilise un procédé d'émulsification agitée comme le procédé d'émulsification de l'étape d'émulsification secondaire.

**7.** Procédé de production de liposomes unilamellaires par moyen d'un procédé d'émulsification en deux étapes selon l'une quelconque des revendications 1 à 5, qui utilise un procédé d'émulsification à micro-canal comme le procédé d'émulsification de l'étape d'émulsification secondaire.

**8.** Procédé de production de liposomes unilamellaires par moyen d'un procédé d'émulsification en deux étapes selon l'une quelconque des revendications 1 à 5, qui utilise un procédé d'émulsification à membrane utilisant une membrane SPG comme le procédé d'émulsification de l'étape d'émulsification secondaire.

**9.** Procédé de production de liposomes unilamellaires par moyen d'un procédé d'émulsification en deux étapes selon l'une quelconque des revendications 1 à 8, dans lequel des médicaments pour des traitements médicaux sont utilisés comme substances destinées à être encapsulées dans les liposomes unilamellaires.

**10.** Procédé de production d'une dispersion de liposomes unilamellaires ou d'une poudre sèche de celle-ci, comprenant le procédé de production de liposomes unilamellaires par moyen d'un procédé d'émulsification en deux étapes selon l'une quelconque des revendications 1 à 9.

**11.** Procédé de production d'une dispersion de liposomes unilamellaires ou d'une poudre sèche de celle-ci selon la revendication 10, qui comprend en outre une étape de séparation pour séparer les liposomes unilamellaires obtenues par l'étape d'émulsification secondaire et l'agent de dispersion les uns de l'autre.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0136583 A1

- WO 2005053643 A

**Non-patent literature cited in the description**

- **TAKASHI KUROIWA ; MITSUTOSHI NAKAJIMA ; SOSAKU ICHIKAWA.** Influences of Aqueous Phase Composition in Lipid Capsule Formation Using Multiphase Emulsion as Base. *Summary of the 74th Annual meeting of the Society of Chemical Engineers,* March 2009